Europäisches Patentamt

(19) **European Patent Office**

Office européen des brevets

(11) Numéro de publication: **0 133 393**
**B1**

(12) **FASCICULE DE BREVET EUROPEEN**

(45) Date de publication du fascicule du brevet:
**16.09.87**

(21) Numéro de dépôt: **84401507.3**

(22) Date de dépôt: **18.07.84**

(51) Int. Cl.⁴: **A 61 F 2/30,** A 61 B 17/28,
A 61 B 19/02

(54) **Dispositif de conditionnement et de manipulation pour un objet devant rester à l'abri d'un contact manuel direct, et ensemble comprenant un tel dispositif et un tel objet.**

(30) Priorité: **02.08.83 FR 8312717**

(43) Date de publication de la demande:
**20.02.85 Bulletin 85/8**

(45) Mention de la délivrance du brevet:
**16.09.87 Bulletin 87/38**

(84) Etats contractants désignés:
**AT BE CH DE GB IT LI LU NL SE**

(56) Documents cités:
**FR - A - 2 405 872**
**US - A - 3 857 389**

(73) Titulaire: **SYNTHESE ET CREATION, 13, blvd des Martyrs de la Résistance, F-04300 Forcalquier (FR)**

(72) Inventeur: **Thomas, Jacques, La Folastière, F-04300 St. Maime (FR)**
Inventeur: **Raval, Claude, Rue Scarpée, F-04300 Dauphin (FR)**

(74) Mandataire: **Martin, Jean-Jacques et al, Cabinet REGIMBEAU 26, Avenue Kléber, F-75116 Paris (FR)**

Il est rappelé que: Dans un délai de neuf mois à compter de la date de publication de la mention de la délivrance du brevet euro-péen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un dispositif de conditionnement et de manipulation pour un objet devant rester à l'abri d'un contact manuel direct, ainsi qu'un ensemble comprenant un tel dispositif et un tel objet.

Elle peut trouver son application notamment dans le conditionnement et la manipulation des prothèses implantables, telles que par exemple les têtes prothétiques de fémur, ceci ne constituant toutefois qu'un exemple non limitatif.

On sait que de telles prothèses sont conditionnées dans des conditions stériles, et doivent être autant que possible conservées dans de telles conditions jusqu'à leur implantation sur un patient.

On sait réaliser des conditionnements propres à autoriser une stérilisation d'un objet conditionné et le maintien de cet objet dans des conditions stériles, et l'on utilise par exemple à cet effet des sachets ou des boîtes étanches.

Le problème se pose toutefois alors de maintenir l'objet conditionné à l'abri de toute souillure lorsque l'on ouvre l'emballage et jusqu'à l'implantation sur le patient, problème que l'on résout en imposant une manipulation au moyen de pinces à quiconque n'est pas muni de gants stériles.

Or, l'extraction d'un objet hors d'un emballage au moyen de pinces n'est pas toujours facile, surtout si cet objet occupe une position indéterminée dans l'emballage, comme c'est le cas par exemple lorsque cet emballage est constitué par un sachet, et si cet objet offre peu de prise; cet inconvénient est d'autant plus sensible que l'objet doit être pris en charge par la pince dans une position relative plus précise, ce qui est le cas par exemple d'objets devant subir une opération d'assemblage après qu'on les a sortis de leur conditionnement, et notamment de certaines prothèses; par exemple, si l'objet considéré est une tête prothétique de fémur, il est nécessaire d'assembler cette tête à une broche de fixation sur le fémur préalablement implantée sur le patient, opération particulièrement délicate si l'on ne bénéficie pas d'une prise convenable sur la tête prothétique, dont la forme essentiellement sphérique constitue précisément un obstacle à une bonne préhension.

Il n'est par conséquent pas rare que l'objet soit pris directement en main par des personnes qui ne sont pas habilités à le faire, et par exemple par un assistant démuni de gants stériles dans le cas d'une prothèse, ce qui peut naturellement ruiner toutes les précautions prises par ailleurs.

Ce problème d'éviter un contact manuel direct avec un objet conditionné n'est pas limité au seul cas des objets stérilisés et l'on peut notamment citer le cas d'objets munis d'un traitement de surface tel qu'un revêtement qu'un contact manuel peut endommager, comme par exemple certaines pièces de mécanique de précision ou d'optique, ou encore certains composants électroniques.

Le but de la présente invention est de proposer un dispositif de conditionnement et de manipulation permettant de remédier à ces inconvénients.

A cet effet, la présente invention propose un dispositif de conditionnement et de manipulation pour un objet devant rester à l'abri d'un contact manuel direct, tel qu'une prothèse implantable, qui comporte:

une pince présentant au moins deux mors mobiles l'un par rapport à l'autre pour évoluer entre un état de fermeture, dans lequel ils sont relativement rapprochés et définissent complémentairement un logement propre à retenir l'objet par conjugaison de formes, et un état d'ouverture dans lequel ils sont relativement écartés pour ouvrir ledit logement et en libérer l'objet, ladite pince présentant en outre, à distance desdits mors, des moyens de manipulation en liaison fonctionnelle avec ces derniers pour provoquer à volonté ladite évolution,

une boîte comportant un récipient et un couvercle, et susceptible de recevoir intérieurement ladite pince dans une position dans laquelle les mors, à l'état de fermeture, et l'objet retenu dans le logement de ces derniers sont placés dans le récipient et les moyens de manipulation tournés vers le couvercle, et en ce que les mors de la pince et le récipient présentent des formes complémentaires telles que ce dernier retienne alors les mors à l'état de fermeture.

On est ainsi assuré de ce que l'objet conserve à tout moment une position parfaitement déterminée par rapport à la pince tant que les mors de cette dernière sont fermés.

De façon particulièrement simple, les mors de la pince peuvent être définis par les premières extrémités respectives de deux branches présentant par ailleurs des deuxièmes extrémités définissant les moyens de manipulation, les deux branches étant articulées entre elles dans une zone intermédiaire entre lesdites premières extrémités et lesdites deuxièmes extrémités.

D'autres caractéristiques et avantages du dispositif ressortiront de la description ci-dessous, relative à l'exemple non limitatif d'un tel dispositif destiné au conditionnement et à la manipulation d'une tête prothétique de fémur, ainsi que des dessins annexés qui font partie intégrante de cette description.

Les fig. 1 et 2 montrent des vues de la boîte fermée, respectivement en élévation latérale et en plan.

La fig. 3 illustre, en une vue en perspective, la coopération de la pince fermée sur la tête prothétique de fémur et de la boîte elle-même fermée sur la pince.

La fig. 4 est une vue en perspective montrant la boîte après ouverture et la pince après extraction hors de la boîte et ouverture.

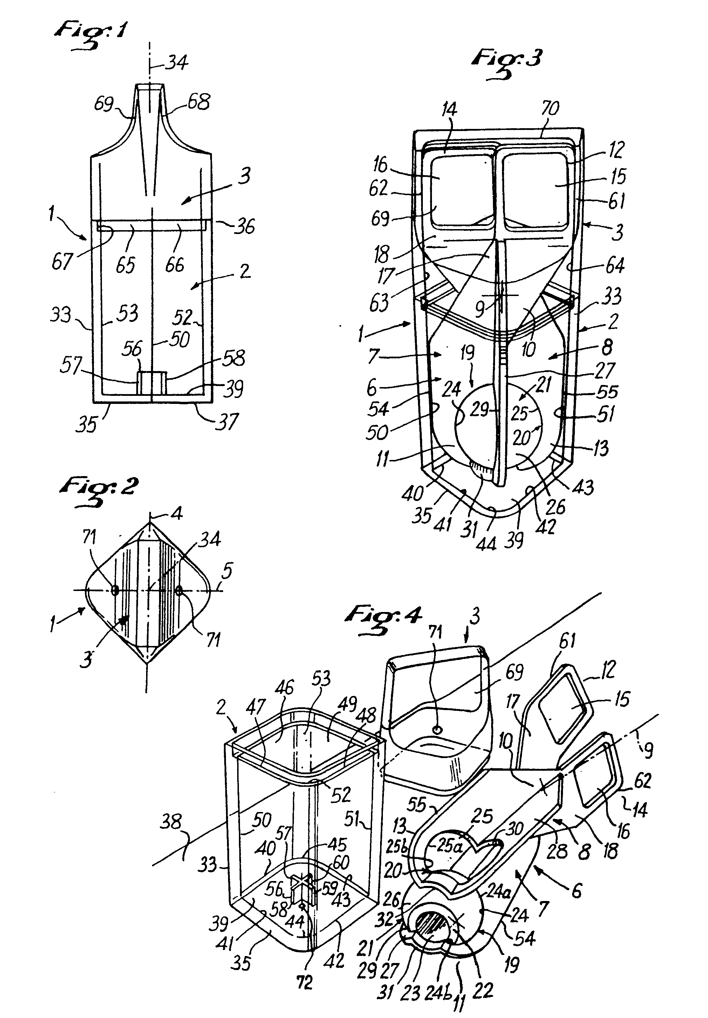

Sur l'ensemble de ces figures, on a désigné de façon générale par 1 la boîte, présentant deux plans de symétrie 4 et 5 perpendiculaires entre eux et formée d'un récipient 2 et d'un couvercle 3 l'un et l'autre symétriques par rapport à ces deux plans, la boîte supposée fermée, telle qu'elle est illustrée aux fig. 1 et 2 par exemple; on a par ailleurs désigné de façon générale par 6 la pince, formée dans cet exemple de deux branches en S 7 et 8 identiques, articulées entre elles autour d'un axe 9 dans une zone de croisement mutuel 10 intermédiaire entre leurs deux zones d'extrémité respectives, 11 et 12 en ce qui concerne la branche 7, 13 et 14 en ce qui concerne la branche 8; le mode d'articulation mutuel des branches 7 et 8 dans leur zone intermédiaire 10 de croisement n'a pas été détaillé; tout moyen du domaine des connaissances de l'homme du métier peut être utilisé pour réaliser cette articulation, pourvu qu'il matérialise l'axe 9 et l'on peut par exemple avoir recours à cet effet soit à une pièce supplémentaire formant pivot d'articulation mutuelle des branches, soit, dans le cas d'une réalisation des branches 7 et 8 en une matière plastique, à un pont de matière plus souple que ces branches essentiellement rigides mais réalisé d'une seule pièce avec elles, l'axe 9 pouvant alors être défini directement par le pont de matière ou par des moyens de guidage mutuel prévus sur les branches, comme il est connu en soi.

La pince 6 va à présent être décrite plus en détail, en référence aux fig. 3 et 4 qui la montrent respectivement à l'état fermé et à l'état ouvert.

On remarque que dans chacun de ces états, les zones d'extrémité 12 et 14 se jouxtent de même que les zones d'extrémité 11 et 13, étroitement lorsque la pince est fermée comme il est illustré à la fig. 3 ou moyennant un écartement relatif lorsque la pince est ouverte comme elle est illustrée à la fig. 4, et qu'un mouvement d'écartement relatif ou de rapprochement relatif imprimé aux zones d'extrémité 12 et 14 des branches 7 et 8 se traduit par un mouvement analogue, respectivement d'éloignement relatif ou de rapprochement relatif, des zones d'extrémité 11 et 13.

En vue de faciliter la préhension manuelle de la pince et sa manœuvre dans le sens d'un écartement ou d'un rapprochement des deux zones d'extrémité 11 et 13, chacune des zones d'extrémité 12 et 14 se présente sous la forme d'un œillet propre à permettre l'engagement d'un doigt de l'utilisateur; cet œillet est avantageusement défini par un trou, respectivement 15 ou 16, traversant de part en part, suivant une orientation parallèle à l'axe 9, une zone d'extrémité, définissant respectivement la zone d'extrémité 12 de la branche 7 ou la zone d'extrémité 14 de la branche 8, d'une plaque plate qui présente selon un plan moyen perpendiculaire à l'axe 9, une forme de S en plan, et qui définit la majeure partie de la branche correspondante de la pince 6; cette partie de branche en forme de plaque a été désignée par la référence 17 en ce qui concerne la branche 7, 18 en ce qui concerne la branche 8.

Par une autre extrémité, la plaque 17 ou 18 définit une partie de l'autre zone d'extrémité, respectivement 11 ou 13, de la branche correspondante de pince et, dans une zone intermédiaire entre ses zones d'extrémité, elle se juxtapose étroitement, à plat, à la partie en forme de plaque de l'autre branche pour définir la zone 10 précitée de croisement mutuel et d'articulation des deux branches 7 et 8.

Dans sa zone d'extrémité correspondant respectivement à la zone d'extrémité 11 de la branche 7 ou à la zone d'extrémité 13 de la branche 8, la plaque 17 ou 18 définit partiellement un mors, respectivement 19 ou 20, de la branche correspondante, les deux mors 19 et 20 étant placés en vis-à-vis et définissant conjointement un logement propre à retenir par complémentarité de forme une tête prothétique de fémur 21 lorsque la pince est à l'état fermé (fig. 3), ce logement étant par contre ouvert pour libérer la tête prothétique 21 lorsque la pince est à l'état ouvert (fig. 4).

Compte tenu de la forme de la tête prothétique 21 choisie à titre d'exemple non limitatif, à savoir la forme générale d'une sphère présentant un méplat circulaire 22 percé centralement d'un borgne 23, de forme générale cylindrique de révolution autour d'un axe radial par rapport au centre de la sphère, en vue de la fixation de cette dernière sur une broche de liaison avec le fémur d'un patient, la plaque 17 ou 18 présente à cet effet dans la zone d'extrémité 11 ou 13 une découpe, respectivement 24 ou 25, qui, vue en plan, présente une zone en forme d'arc de cercle, respectivement 24a ou 25a, propre à s'appliquer contre la partie sphérique 26 de la périphérie extérieure de la tête prothétique 21, selon un méridien de cette partie 26, et une zone, respectivement 24b ou 25b, rectiligne et propre à s'appliquer contre le méplat 22, selon un rayon de ce dernier, entre la jonction de ce méplat 22 avec la partie sphérique 26 et le trou borgne 23 en laissant ce dernier totalement dégagé.

Chacune des zones 24a et 25a présente un développement angulaire intermédiaire entre 90° et 180° et les découpes 24 et 25 sont disposées, respectivement sur la branche 17 et sur la branche 18, de telle sorte qu'elles soient en vis-à-vis et que, lorsque la pince est à l'état fermé, les zones 24a et 25a se complètent en un arc de cercle unique entourant la partie sphérique 26 de la tête 21 sur plus de 180°, ce qui empêche alors tout mouvement de la tête 21 parallèlement aux plans moyens respectifs des plaques 17 et 18; les zones 24b et 25b des découpes 24 et 25 empêchent quant à elles toute rotation de la tête 21 autour d'un axe parallèle à l'axe 9; elles constituent les zones respectives des découpes 24 et 25 les plus éloignées de ce dernier, de telle sorte que le trou borgne 23 de la sphère 21 s'ouvre vers l'extrémité de la pince 6.

En outre, afin que la tête prothétique 21 soit retenue dans son logement, lorsque la pince 6 est fermée, également à l'encontre de tout mouvement dans des plans perpendiculaires aux plans moyens respectifs des plaques 17 et 18, ces dernières portent de façon solidaire, de leur zone intermédiaire définissant la zone de croisement mutuel 10 à leur zone extrême définissant la zone d'extrémité 11 ou 13 de la branche correspondante, respectivement 7 ou 8, une aile latérale respective 27 ou 28 qui peut avantageusement être réalisée en une pièce avec la plaquette correspondante 17 ou 18.

L'aile 27 est plate et présente un plan moyen incluant l'axe 9 ou parallèle à ce dernier à proximité immédiate de lui, et perpendiculaire au plan moyen de la plaque 17; dans la zone d'extrémité 11 de la branche 7, elle présente une découpe 29 identique à la découpe 24 de la plaque 17, c'est-à-dire présentant, lorsqu'elle est vue en plan, une zone en forme d'arc de cercle propre à épouser sur plus de 90° et moins de 180° un méridien de la surface sphérique 26 et une zone, relativement plus éloignée de l'axe 9, rectiligne pour s'appliquer sur le méplat 22 entre sa jonction avec la surface sphérique 26 et le trou borgne 23 sans entraver l'accès à ce dernier lorsque la pince est fermée sur la tête prothétique 21; de même, l'aile 28 présente dans la zone d'extrémité 13 de la branche 10 une découpe 30 en tout point identique à la découpe 25, c'est-à-dire présentant, lorsqu'elle est vue en plan, une zone relativement plus proche de l'axe 9 est présentant la forme d'un arc de cercle propre à épouser la surface 26 sur plus de 90° et moins de 180°, et une zone relativement plus éloignée de l'axe 9 et présentant une forme rectiligne pour épouser le méplat 22 entre la surface 26 et le trou borgne 23.

Lorsque la pince est fermée, les parties respectives des découpes 24, 25, 29, 30 correspondant à des arcs de cercle sont disposées selon une même surface sphérique complémentaire de la surface 26, et les zones de ces découpes qui présentent une forme en plan rectiligne sont alors coplanaires, selon un plan coïncidant avec celui du méplat 22; on remarquera que, pour améliorer la rigidité de chacune des branches 7 et 8 de la pince en améliorant l'appui de ces branches sur le méplat 22, la zone de l'aile 27 la plus éloignée de l'axe 9, dans la zone d'extrémité 11 de la branche 7 de la pince, est reliée à la partie correspondante de la plaque 17 par un secteur 31 présentant vers l'intérieur du logement ainsi défini pour la tête prothétique 21 une face plane, coplanaire avec la zone 24b de la découpe 24 et la zone correspondante de la découpe 29, et que l'aile 28 et la plaque 18 sont reliées de façon identique, dans la zone d'extrémité 13 de la branche 8, par un sectur 32 présentant vers l'intérieur du logement pour la tête prothétique 21 une face coplanaire avec la zone 25b de la découpe 25 et la zone correspondante de la découpe 30; lorsque la pince est fermée, ces secteurs 31 et 32 laissent dégagé le trou borgne 23, qu'ils entourent partiellement.

De préférence, la surface de contact entre les découpes 24, 25, 29, 30 et les zones correspondantes de la périphérie extérieure de la tête prothétique 21 sont aussi limitées que possible et, à cet effet, on peut donner aux plaques 17 et 18 et aux ailes 27 et 28, au niveau des découpes respectives, la forme d'arétes propres à assurer un contact linéaire, et/ou une forme s'écartant par zone de la stricte complémentarité vis-à-vis de la forme de la surface 26 ou de celle du méplat 22 pour assurer un contact proche d'un contact ponctuel avec la tête prothétique 21.

Ainsi, celle-ci peut être stérilisée aussi complètement que possible alors qu'elle est placée et immobilisée dans la pince elle-même placée et immobilisée dans la boîte 1, grâce aux dispositions complémentaires de cette dernière qui vont être décrites à présent.

Il ressort des figures que le récipient 2 se présente sous la forme d'un manchon tubulaire 33 qui présente un axe 34 défini par l'intersection des plans de symétrie 4 et 5, et est fermé à l'une de ses extrémités par un fond plat 35 perpendiculaire à cet axe 34 alors qu'il est ouvert à son autre extrémité, en 36; avantageusement, le manchon 33 et son fond 35 sont réalisés en une seule pièce d'une matière plastique apte à être stérilisée.

A l'extérieur du récipient 2, le fond 35 présente une face plane 37 perpendiculaire à l'axe 34 et par laquelle le récipient 2 éventuellement muni du couvercle 3 peut être posé sur la face supérieure plane, horizontale 38 d'un support quelconque; à l'intérieur du récipient, ce fond 35 présente également une face plane 39 perpendiculaire à l'axe 34, laquelle face présente la forme générale d'un carré définie par quatre côtés rectilignes 40, 41, 42, 43, parallèles deux à deux, à raison de deux côtés 40 et 41 se coupant à angle droit sur le plan 4, deux côtés 40 et 41 se coupant à angle droit sur le plan 4, deux côtés 42 et 43 se coupant également à angle droit sur le plan 4, alors que les côtés 41 et 42 se raccordent entre eux par un arrondi 44 coupant le plan 5, de même que les côtés 40 et 41 se raccordent par un arrondi 45 à l'intersection de ce plan.

Vers l'intérieur du récipient 2, le manchon tubulaire 33 présente quant à lui une face périphérique dont les génératrices sont parallèles à l'axe 34 et dont la directrice est constituée par la périphérie extérieure de la face 39 du fond 35, et présente de ce fait quatre zones planes 46 à 49, correspondant respectivement aux côtés 40 à 43 de la face 39, les zones 46 et 47 se coupant dans le plan 4 en définissant une arête 50 rectiligne et parallèle à l'axe 34 et les zones 48 et 49 se coupant également dans ce plan 4 le long d'une arête 51 rectiligne et parallèle à l'axe 34, alors que les zones 47 et 48 d'une part, 46 et 49 d'autre part sont reliées par les zones arrondies, respectivement 52 et 53 chevauchant le plan 5 et correspondant respectivement aux arrondis 44 et 45 de la face 39 du fond 35; cette face périphérique s'interrompt à proximité immédiate de l'ouverture 36, selon un plan perpendiculaire à l'axe 34, et présente sur la totalité de la périphérie de l'ouverture 36 un décrochement 67 dans le sens l'un éloignement par rapport à l'axe 34 et dont l'utilité apparaîtra plus loin.

On remarquera qu'ainsi, le manchon tubulaire 33 du récipient 2 présente dans le plan 4 sa plus grande dimension intérieure transversalement par rapport à l'axe 34; notamment, cette dimension est supérieure à la dimension correspondante dans le plan 5.

Parallèlement à l'axe 34, le manchon tubulaire 33 présente, entre la face 39 du fond 35 et l'ouverture 36, une longueur du même ordre que la distance séparant de l'axe 9 la partie des zones d'extrémité respectives 11 et 13 des branches 7 et 8 qui est la plus éloignée de cet axe 9 et, sur la majeure partie des zones d'extrémité respectives 11 et 13 des branches correspondantes, respectivement 7 et 8, les plaques 17 et 18 de ces dernières présentent à l'opposé de la découpe 24, 25, c'est-à-dire vers l'extérieur du logement défini pour la tête prothétique 21 par les mors 19 et 20, des arêtes respectives 54 et 55 qui sont rectilignes, parallèles entre elles lorsque la pince est à l'état fermé, et distantes mutuellement d'une distance inférieure à la distance séparant les arêtes 50 et 51 mais suffisamment proche de celle-ci pour que la pince, à l'état fermé, puisse être introduite dans le récipient 2 ou extraite de ce dernier via l'ouverture 36 moyennant un coulissement des arêtes 54 et 55 contre ou pratiquement contre les arêtes 50 et 51, par un déplacement relatif de la pince 6 et du récipient 2 parallèlement à l'axe 34 de ce dernier, et que le contact entre d'arête 54 et l'arête 50 d'une part, l'arête 55 et l'arête 51 d'autre part interdise l'ouverture de la pince dès lors que les zones d'extrémité 11 et 13 des branches 7 et 8 de celle-ci sont ainsi engagées dans le récipient 2; les arêtes 54 et 55 sont reliées au secteur d'extrémité, respectivement 31 ou 32, de la branche correspondante par une arête curviligne, convexe, intégralement située en retrait par rapport à elles pour autoriser et faciliter l'introduction de la pince 6, fermée, dans le récipient 2.

On est ainsi assuré de ce que la tête prothétique 21 reste emprisonnée entre les mors 19 et 20 que constituent respectivement dans la zone l'extrémité 11 de la branche 7 et dans la zone d'extrémité 13 de la branche 8 les découpes 24–29 et 25–30, respectivement.

La distance séparant les arêtes 54 et 55 définit l'encombrement hors tout maximal de la juxtaposition des zones d'extrémités 11 et 13 des branches 7 et 8 de la pince 6 à l'état fermé et de la tête prothétique 21 que renferme celle-ci, cet encombrement étant mesuré perpendiculairement à un axe se confondant avec l'axe 34 lorsque la pince est engagée dans le récipient 2 par les zones d'extrémité 11 et 13.

On remarquera qu'une introduction de la pince fermée dans le récipient 2 selon une orientation relative, autour de l'axe 34, correspondant à placer les arêtes 54 et 55 respectivement en regard des arêtes 51 et 50 serait possible, mais que tout autre mode d'introduction serait impossible, la forme intérieure du manchon tubulaire 33 comparée à la distance séparant les arêtes 54 et 55 lorsque la pince est fermée amenant ainsi un détrompage efficace.

Le cas échéant, on peut prévoir que les ailes respectives 27 et 28 des branches 7 et 8 présentent également, vers l'extérieur par rapport au logement de la tête prothétique 21, des arêtes rectilignes s'orientant parallèlement entre elles lorsque la pince est fermée, à la condition que la distance séparant alors ces arêtes soit inférieure à la distance séparant les zones 52 et 53 dans le plan 5.

Pour améliorer l'immobilisation de la tête prothétique 21 non seulement par rapport à la pince 6 mais également par rapport au récipient 2 lorsque, lors de l'introduction de la pince 6 portant une tête prothétique 21 dans le récipient 2, cette dernière parvient à proximité de la face 39 du fond 35 de celui-ci, cette face 39 est munie centralement, c'est-à-dire suivant l'axe 34, d'un relief pyramidal 56 dont la forme peut varier dans une large mesure, et qui présente dans l'exemple illustré la forme d'un croisillon d'axe 34, délimité dans le sens d'un éloignement par rapport à ce dernier par des arêtes 57 à 60 par lesquelles il s'emmanche dans le trou borgne 23 de la tête prothétique 21 à la fin du processus d'introduction de la pince 6 fermée dans le récipient 2 par déplacement relatif parallèlement à l'axe 34; naturellement, le relief 56 pourrait également présenter une autre forme, celle-ci étant toutefois choisie de préférence de telle façon que, lors de la stérilisation, le trou borgne 23 puisse également être traité; à cet effet, on remarquera que c'est à proximité immédiate du relief 56, et plus précisément entre les branches du croisillon que forme ce dernier dans l'exemple illustré, que l'on a aménagé dans le fond 35 du récipient 2 un ou plusieurs trous 72 de passage de vapeur, ouverts pendant la stérilisation puis ensuite obturés de façon étanche par exemple au moyen d'un ruban adhésif.

Compte tenu de la dimension, indiquée plus haut, que le récipient 2 présente intérieurement parallèlement à l'axe 34, l'introduction de la pince 6 au maximum dans le récipient 2 amène l'articulation 9 approximativement au niveau de l'ouverture 36, et les zones d'extrémité 12 et 14 des branches 7 et 8, c'est-à-dire les œillets définis par les trous 15 et 16, restent en dehors du récipient 2, ce qui facilite considérablement la préhension de la pince 6.

Toutefois, lorsque la boîte est fermée, ces zones 12 et 14 sont intégralement englobées par le couvercle 3 qui, lorsqu'il est en place sur le récipient 2, ferme ce dernier de façon étanche et constitue en outre, avantageusement, une butée s'opposant à tout déplacement de la pince 6, et de la tête prothétique 21 qu'elle porte, dans le sens d'un éloignement par rapport au fond 35 du récipient 2 suivant l'axe 34.

En outre, les zones d'extrémité respective 12 et 14 des branches 7 et 8 de même que le couvercle 3 sont avantageusement conçus de telle sorte que la mise en place du couvercle sur le récipient 2 emprisonne étroitement ces zones 12 et 14 pour contribuer ainsi au maintien de la pince à l'état fermé.

A cet effet, les zones 12 et 14 présentent des arêtes rectilignes, respectivement 61 et 62, qui se placent parallèlement entre elles et respectivement dans le prolongement de l'arête 55 et dans celui de l'arête 54 lorsque la pince est fermée, en définissant ainsi la plus grande dimension de cette pince au niveau des zones d'extrémité 12 et 14, transversalement par rapport à un axe se confondant avec l'axe 34 lorsque la pince 6, fermée, est engagée dans le récipient 2, et le couvercle 3 présente des arêtes intérieures rectilignes, parallèles 63 et 64 qui se placent dans le prolongement direct respectivement de l'arête 50 et de l'arête 51 lorsqu'il est mis en place sur le récipient 2, si bien que ces arêtes 63 et 64 se placent alors en regard des arêtes 62 et 61, respectivement, pour contribuer à empêcher l'ouverture de la pince; on remarquera que celle-ci, à l'état fermé, de même que la tête prothétique 21 qu'elle retient, sont intégralement situées entre les arêtes 61 et 55 d'une part et les arêtes 62 et 54 d'autre part.

Un mode de liaison entre le couvercle 3 et le récipient 2 compatible avec une telle coopération est plus particulièrement visible aux fig. 1 et 3, où l'on voit que les arêtes 63 et 64 se prolongent jusqu'à une ouverture 65 du couvercle 3, laquelle est bordée d'un rebord continu 66 apte à s'emboîter de façon étanche à l'intérieur de décrochement 67 que le manchon tubulaire 33 présente intérieurement autour de l'ouverture 36; les arêtes 63 et 64 se prolongent jusqu'à la limite du rebord 66 la plus extérieure au couvercle 3.

Dans ses zones adjacentes à l'ouverture 65, le couvercle 3 présente une face périphérique intérieure prolongeant exactement celle du manchon 33, mais cette face périphérique intérieure se rétrécit ensuite, dans le sens d'un éloignement par rapport à l'ouverture 65, de telle sorte que le couvercle 3 présente intérieurement, dans sa zone la plus éloignée de l'ouverture 65, la forme d'une fente disposée selon le plan 4 avec des dimensions telles qu'il enserre à ce niveau les zones d'extrémité 12 et 13 des branches 7 et 8 non seulement par leurs arêtes 61 et 62, grâce aux arêtes 63 et 64, mais également perpendiculairement aux plans moyens respectifs des plaques 17 et 18; cette zone du couvercle 3 a été désignée par la référence 68 aux figures; on remarquera que, le couvercle 3 présentant une épaisseur de paroi sensiblement constante, cette zone rétrécie 68 donne lieu, à l'extérieur du couvercle 3, à l'existence d'une zone aplatie 69 facilitant considérablement la préhension de ce couvercle lors de sa mise en place sur le récipient 2 ou de son enlèvement.

Par ailleurs, le couvercle 3 est avantageusement muni d'orifices 71, permettant une stérilisation correcte. Ces orifices sont obturés à l'aide de tous moyens appropriés après l'opération de stérilisation. De préférence ils sont ménagés sur la portion du couvercle généralement parallèle au fond 35 du récipient 2.

Dans le sens d'un éloignement apr rapport à l'ouverture 65, la zone rétrécie 68 de la face périphérique intérieure de ce couvercle se termine par jonction avec une face plane 70 perpendiculaire à l'axe 34, laquelle sert de butée aux zones d'extrémité 12 et 14 des branches 7 et 8 de la pince 6 pour empêcher tout mouvement de cette dernière dans le sens d'un dégagement vis-à-vis du récipient 2 lorsqu'elle est engagée à fond dans ce dernier; l'homme du métier déterminera aisément les dimensions intérieures données à cet effet au couvercle 3, compte tenu des dimensions de la pince 6.

Le mode d'utilisation du dispositif qui vient d'être décrit peut être le suivant: sur les lieux de fabrication, on place la tête prothétique 21 convenablement orientée dans l'un des mors de la pince ouverte, puis on ferme celle-ci et on l'engage, par les zones d'extrémité 11 et 13, dans le récipient 2 via l'ouverture 36, en respectant l'orientation angulaire relative imposée, en référence à l'axe 34, par la forme intérieure du récipient 2 et par celle de la pince; à la fin de cette introduction, la tête prothétique 21 s'emboîte sur le relief 56 du fond 35 du récipient 2; on peut alors mettre en place le couvercle 3, en l'orientant convenablement en regard à sa zone rétrécie 68, ce à quoi contribuent d'ailleurs les formes complémentaires du rebord 66 et de l'épaulement 67, lesquels présentent des faces respectivement extérieure.et intérieure homothétiques de la périphérie intérieure du manchon 33 et constituant de ce fait des moyens de détrompage; le couvercle 3 est ainsi emboîté à fond sur le récipient 2 par mouvement relativ parallèlement à l'axe 34, et on peut procéder à la stérilisation de l'ensemble formé par la boîte 1, la pince 6 et la tête prothétique 21, puis obturer le trou ou les trous 61 pour fermer la boîte 1 de façon étanche.

A l'utilisation, par préhension dans la zone 69, un assistant du chirurgien ôte le couvercle 3, ce qui dégage les zones d'extrémité 12 et 14 de la pince 6, par lesquelles le chirurgien peut prendre directement cette dernière de façon particulièrement commode et l'utiliser pour placer la tête prothétique sur la broche préalablement implantée. En cas de problème le chirurgien peut réextraire la tête prothétique de la même manière et la replacer dans le récipient 2 toujours maintenu par l'assistant sans risque de contamination à aucun moment. La conformation parfaitement rigide et robuste de la pince, et permettant en particulier de maintenir la tête dans une position déterminée avec précision permet au chirurgien de réaliser l'emboitement en force de la tête prothétique sur la broche sans intervention directe des mains. L'ouverture de la pince libère la tête prothétique quand la fixation définitive est réalisée.

On comprendra que, moyennant une conformation appropriée des mors de la pince, le mode de conditionnement qui vient d'être décrit puisse être adapté au conditionnement et à la manipulation de prothèses autres que des têtes de fémur, et de façon plus générale aux objets les plus divers dès lors qu'il y a lieu de les protéger d'un contact manuel direct; le mode d'utilisation du dispositif peut être dans ces différents cas analogue à celui qui vient d'être décrit, si ce n'est naturellement qu'il n'y a pas stérilisation lorsqu'une telle stérilisation n'est pas requise.

On notera par ailleurs, lorsque des séries d'objets différents entre eux sont conditionnés dans des emballages identiques qu'il est avantageux de prévoir un codage sur les pinces afin de distinguer visuellement ces objets. Un tel codage pourra par exemple être obtenu par coloration prédéterminée des pinces, les récipients étant quant à eux transparents.

**Revendications**

1. Dispositif de conditionnement et de manipulation pour un objet (21) devant rester à l'abri d'un contact manuel direct, tel qu'une prothèse implantable, qui comporte:

une pince (6) présentant au moins deux mors (19, 20) mobiles l'un par rapport à l'autre pour évoluer entre un état de fermeture, dans lequel ils sont relativement rapprochés et définissent complémentairement un logement (24, 25, 29, 30) propre à retenir l'objet (21) par conjugaison de formes, et un état d'ouverture dans lesquels ils sont relativement écartés pour ouvrir ledit logement (24, 25, 29, 30) et en libérer l'objet (21), ladite pince (6) présentant en outre, à distance desdits mors (19, 20), des moyens de manipulation (12, 14, 15, 16) en liaison fonctionnelle avec ces derniers pour provoquer à volonté ladite évolution,

une boîte (1) comportant un récipient (2) et un couvercle (3), et susceptible de recevoir intérieurement ladite pince (6) dans une position dans laquelle les mors (19, 20), à l'état de fermeture, et l'objet (21) retenu dans le logement (24, 25, 29, 30) de ces derniers sont placés dans le récipient (2) et les moyens de manipulation (12, 14, 15, 16) tournés vers le couvercle (3),

et en ce que les mors (19, 20) de la pince (6) et le récipient (2) présentent des formes complémentaires telles que ce dernier retienne alors les mors (19, 20) à l'état de fermeture.

2. Dispositif selon la revendication 1, caractérisé en ce que les mors (19, 20), au nombre de deux, sont définis par les premières extrémités respectives (11, 13) de deux branches (7, 8) présentant par ailleurs des deuxièmes extrémités (12, 14) définissant les moyens de manipulation (15, 16), les deux branches (7, 8) étant articulées entre elles dans une zone (10) intermédiaire entre lesdites premières extrémités (11, 13) et lesdites deuxièmes extrémités (12, 14).

3. Dispositif selon l'une quelconque des revendications 1 et 2, caractérisé en ce que le couvercle (3) est creux, et en ce que, dans une position d'engagement maximal de la pince (6) dans le récipient (2), les moyens de manipulation (12, 14, 15, 16) sont placés à l'extérieur de ce dernier, dans le couvercle (3).

4. Dispositif selon l'une quelconque des revendications 1 à 3, caractérisé en ce que le récipient (2) intérieurement et les mors (19, 20), à l'extérieur du logement (24, 25, 29, 30), présentent des moyens complémentaires (50, 51, 54, 55) de guidage a'u coulissement relatif selon une direction (34) déterminée lorsque les mors (19, 20) sont à l'état de fermeture, et de butée mutuelle à l'encontre d'un passage de ces derniers à l'état d'ouverture, et en ce que le couvercle (3) présente des moyens de butée (70) propres à s'opposer, lorsqu'il ferme le récipient (2), à tout coulissement de la pince (6) dans le sens d'un dégagement par rapport à celui-ci, selon ladite direction (34), à partir d'une position d'engagement maximal de la pince (6) dans le récipient (2).

5. Dispositif selon l'une quelconque des revendications 1 à 4, caractérisé en ce que le récipient (2) présente intérieurement un relief (56) partiellement complémentaire de celui de l'objet (21) pour contribuer à immobiliser celui-ci dans une position d'engagement maximal de la pince (6) dans le récipient (2).

6. Dispositif selon les revendications 4 et 5 en combinaison, l'objet (21) présentant un trou (23), caractérisé en ce que les mors (19, 20) définissent ledit logement (24, 25, 29, 30) de telle sorte qu'il retienne l'objet (21), à l'état de fermeture des mors (19, 20), dans une position dans laquelle ledit trou (21) est tourné à l'opposé des moyens de manipulation (12, 14, 15, 16), selon ladite direction (34), et laisse ce trou (23) dégagé, et en ce que le récipient (2) présente un fond (35) transversal par rapport à ladite direction (34) et, sur ce fond (35), intérieurement un relief pyramidal (56) complémentaire dudit trou (23) et sur lequel l'objet (21) s'emboîte par ce dernier dans ladite position d'engagement maximal de la pince (6) dans le récipient (2).

7. Dispositif selon les revendications 2 et 3 en combinaison avec l'une quelconque des revendications 4 à 6, caractérisé en ce que le couvercle (3), intérieurement, et lesdites deuxièmes extrémités (12, 14) présentent des moyens complémentaires (61, 62, 63, 64, 68) de guidage au coulissement relatif selon ladite direction (34) lorsque les mors (19, 20) sont à l'état de fermeture, et de butée mutuelle à l'encontre d'un mouvement relatif de ces deuxièmes extrémités (12, 14) correspondant à un passage des mors (19, 20) à l'état d'ouverture.

8. Dispositif selon l'une quelconque des revendications 1 à 7, caractérisé en ce que la boîte (1) est étanche.

9. Ensemble formé par un dispositif selon la revendication 8 et par une prothèse implantable constituant ledit objet (21), caractérisé en outre en ce qu'il est stérilisable.

10. Ensemble selon la revendication 9 dans sa dépendance vis-à-vis de l'une quelconque des revendications 6 et 7, ladite prothèse (21) étant une tête prothétique de fémur présentant ledit trou (23) en vue de son montage sur une broche de fixation.

**Patentansprüche**

1. Vorrichtung zum Verpacken und Handhaben eines Gegenstands (21), der nicht in unmittelbaren manuellen Kontakt gelangen darf, wie etwa eine inplantierbare Prothese, welche aufweist:

eine Zange (6), die mindestens zwei Greifbacken (19, 20) aufweist, welche gegeneinander beweglich sind zum Zweck einer Zustandsänderung zwischen einem Schliesszustand, bei dem sie einander angenähert sind und zusammen eine Aufnahme (24, 25, 29, 30) zur Halterung des Gegenstands (21) durch Formanpassung definieren, und einem Öffnungszustand, bei dem sie auseinandergespreizt sind, um die Aufnahme (24, 25, 29, 30) zu öffnen und den Gegenstand (21) davon freizugeben, wobei die Zange (6) ausserdem im Abstand von den Greifbacken (19, 20) Handhabungsmitteln (12, 14, 15, 16) in Funktionsverbindung mit letzteren aufweist, um die Zustandsänderung wahlweise herbeizuführen,

und eine Schachtel (1) mit einem Behälter (2) und einem Deckel (3), die geeignet ist, in ihrem Inneren die Zange (6) in einer Stellung aufzunehmen, in der sich die Greifbacken (19, 20) im Schliesszustand befinden und der in der Aufnahme (24, 25, 29, 30) gehaltene Gegenstand (21) in dem Behälter (2) angeordnet und die Handhabungsmitteln (12, 14, 15, 16) zum Deckel (3) gerichtet sind, wobei die Greifbacken (19, 20) der Zange (6) und der Behälter (2) komplementäre Formen derart aufweisen, dass letzterer dann die Greifbacken (19, 20) im Schliesszustand hält.

2. Vorrichtung nach Anspruch 1, dadurch gekennzeichnet, dass die Greifbacken (19, 20), zwei an der Zahl, durch die jeweiligen ersten Endabschnitte (11, 13) von zwei Schenkeln (7, 8) gebildet werden, welche ausserdem zweite Endabschnitte (12, 14) aufweisen, welche die Handhabungsmitteln (15, 16) bilden, wobei die zwei Schenkel (7, 8) miteinander in einem Mittelbereich (10) zwischen den ersten (11, 13) und den zweiten Endabschnitten (12, 14) gelenkig verbunden sind.

3. Vorrichtung nach einem der Ansprüche 1 und 2, dadurch gekennzeichnet, dass der Deckel (3) hohl ist und dass in einer Stellung maximalen Einschubs der Zange (6) in dem Behälter (2) die Handhabungsmitteln (12, 14, 15, 16) ausserhalb des Behälters in dem Deckel (3) angeordnet sind.

4. Vorrichtung nach einem der Ansprüche 1 bis 3, dadurch gekennzeichnet, dass der Behälter (2) an der Innenseite und die Greifbacken (19, 20) an der Aussenseite der Aufnahme (24, 25, 29, 30) komplementäre Mitteln zur Führung (50, 51, 54, 55) für eine Relativverschiebung in einer festgelegten Richtung (34), wenn die Greifbacken (19, 20) im Schliesszustand sind, und für einen gegenseitigen Anschlag gegen eine Zustandsänderung der letzteren zu deren Öffnungszustand aufweisen und dass der Deckel (3) Anschlagmitteln (70) aufweist, welche in der Lage sind, wenn der Deckel den Behälter (2) verschliesst, jegliche Verschiebung der Zange (6) im Sinne eines Loslösens von letzterem in der erwähnten Richtung (34) aus der Stellung maximalen Einschubs der Zange (6) in dem Behälter (2) zu verhindern.

5. Vorrichtung nach einem der Ansprüche 1 bis 4, dadurch gekennzeichnet, dass der Behälter (2) im Inneren ein Profil (56) aufweist, welches teilweise zu dem des Gegenstands (21) komplementär ist, um zu einer Fixierung desselben in einer Stellung maximalen Einschubs der Zange (6) in dem Behälter (2) beizutragen.

6. Vorrichtung nach den Ansprüchen 4 und 5, wobei der Gegenstand (21) ein Loch (23) aufweist, dadurch gekennzeichnet, dass die Greifbacken (19, 20) die erwähnte Aufnahme (24, 25, 29, 30) derart bilden, dass sie den Gegenstand (21) im Schliesszustand der Greifbacken (19, 20) in einer Stellung hält, bei der das Loch (23) von den Handhabungsmitteln (12, 14, 15, 16) in der erwähnten Richtung (34) abgewandt ist, und das Loch (23) ausser Eingriff lässt, und dass der Behälter (2) einen Boden (35) quer zur erwähnten Richtung (34) sowie auf dem Boden (35) an der Innenseite eine pyramidenförmige Erhebung (56) aufweist, welche zu dem Loch (23) komplementär ist und auf welcher der Gegenstand (21) in der erwähnten Stellung maximalen Einschubs der Zange (6) in dem Behälter (2) passend sitzt.

7. Vorrichtung nach den Ansprüchen 2 und 3 in Verbindung mit einem der Ansprüche 4 bis 6, dadurch gekennzeichnet, dass der Deckel (3) an der Innenseite und die erwähnten zweiten Endabschnitte (12, 14) komplementäre Mitteln (61, 62, 63, 64, 68) zur Führung bei einer Relativverschiebung in der erwähnten Richtung (34) im Schliesszustand der Greifbacken (19, 20) sowie zum gegenseitigen Anschlag gegen eine Relativbewegung dieser zweiten Endabschnitte entsprechend einem Durchgang der Greifbacken (19, 20) zu deren Öffnungszustand aufweisen.

8. Vorrichtung nach einem der Ansprüche 1 bis 7, dadurch gekennzeichnet, dass die Schachtel (1) dicht abgeschlossen ist.

9. Anordnung aus einer Vorrichtung nach Anspruch 8 und einer implantierbaren Prothese, welche den erwähnten Gegenstand (21) bildet, dadurch gekennzeichnet, dass sie sterilisierbar ist.

10. Anordnung nach Anspruch in Abhängigkeit von einem der Ansprüche 6 und 7, dadurch gekennzeichnet, dass die Prothese (21) ein künstlicher Gelenkkopf für einen Schenkelknochen ist, welcher das erwähnte Loch (23) im Hinblick auf seine Befestigung auf einem Fixierdorn aufweist.

## Claims

1. Packaging and manipulation device for an object (21) not to be handled directly, such as an implantable prosthesis, which comprises:

a gripper (6) comprising at least two jaws (19, 20) able to move one with respect to the other for moving between a closed state in which they are relatively close together and by way of addition define a housing (24, 25, 29, 30) able to retain the object (21) by the pairing of shapes and an open state in which they are relatively apart in order to open the said housing (24, 25, 29, 30) and release the object (21) therefrom, the said gripper (6) also comprising, at a distance from the said jaws (19, 20), manipulation means (12, 14, 15, 16) in functional connection with the latter in order to cause the said movement at will,

a box (1) comprising a container (2) and a lid (3) and able to receive internally the said gripper (6) in a position in which the jaws (19, 20), in the closed state, and the object (21) retained in the housing (24, 25, 29, 30) of the latter are placed in the container (2) and the manipulation means (12, 14, 15, 16) directed towards the lid (3),

and wherein the jaws (19, 20) of the gripper (6) and the container (2) have complementary shapes such that the latter thus retains the jaws (19, 20) in the closed state.

2. Device according to claim 1, characterised in that the jaws (19, 20), of which there are two, are defined by the respective first ends (11, 13) of two arms (7, 8) also comprising second ends (12, 14) defining the manipulation means (15, 16), the two arms (7, 8) being pivoted to each other in an intermediate area (10) between the said first ends (11, 13) and the said second ends (12, 14).

3. Device according to one of claims 1 and 2, characterised in that the lid (3) is hollow and in that, in a position of maximum engagement of the gripper (6) in the container (2), the manipulation means (12, 14, 15, 16) are located outside the latter, in the lid (3).

4. Device according to one of claims 1 to 3, characterised in that the container (2) internally and the jaws (19, 20), outside the housing (24, 25, 29, 30), comprise complementary means (50, 51, 54, 55) for guidance for relative sliding in a predetermined direction (34) when the jaws (19, 20) are in the closed state and for mutual abutment in opposition to a passage of the latter into the open state and in that the lid (3) comprises abutment means (70) able to oppose, when it closes the container (2), any sliding of the gripper (6) in the direction of disengagement with respect to the latter, in the said direction (34), from a position of maximum engagement of the gripper (6) in the container (2).

5. Device according to one of claims 1 to 4, characterised in that internally the container (2) has a relief (56) which partly complements that of the object (21) in order to contribute to immobilising the latter in a position of maximum engagement of the gripper (6) in the container (2).

6. Device according to claims 4 and 5 in combination, the object (21) comprising a hole (23), characterised in that the jaws (19, 20) define the said housing (24, 25, 29, 30) so that it retains the object (21), in the closed state of the jaws (19, 20), in a position in which the said hole (21) is turned away from the manipulation means (12, 14, 15, 16) in the said direction (34) and this hole (23) is left clear and in that the container (2) comprises a transverse base (35) with respect to the said direction (34), and, on this base (35) internally a pyramidal relief (56) complementing the said hole (23) and on which the object (21) fits by the latter in the said position of maximum engagement of the gripper (6) in the container (2).

7. Device according to claims 2 and 3 in combination with one of claims 4 to 6, characterised in that the lid (3), internally and the said second ends (12, 14) comprise complementary means (61, 62, 63, 64, 68) for guidance for relative sliding in the said direction (34) when the jaws (19, 20) are in the closed state and for mutual abutment in opposition to a relative movement of these two ends (12, 14) corresponding to a passage of the jaws (19, 20) into the open state.

8. Device according to one of claims 1 to 7, characterised in that the box (1) is impervious.

9. Arrangement formed by a device according to claim 8 and by an implantable prosthesis constituting the said object (21), also characterised in that it can be sterilised.

10. Arrangement according to claim 9 depending on one of claims 6 and 7, the said prosthesis (21) being a prosthetic femur head comprising the said hole (23) with a view to its mounting on a securing pin.

Fig:1

Fig:2

Fig:3

Fig:4